**Europäisches Patentamt**

**European Patent Office**

**Office européen des brevets**

(19)

(11) Veröffentlichungsnummer: **0 127 093**
**B1**

(12) # EUROPÄISCHE PATENTSCHRIFT

(45) Veröffentlichungstag der Patentschrift:
**10.06.87**

(21) Anmeldenummer: **84105698.9**

(22) Anmeldetag: **18.05.84**

(51) Int. Cl.⁴: **F 01 K 23/06**, F 02 C 3/28,
F 02 C 6/14, C 07 C 29/15

(54) **Mittellastkraftwerk mit einer integrierten Kohlevergasungsanlage.**

(30) Priorität: **31.05.83 DE 3319732**

(43) Veröffentlichungstag der Anmeldung:
**05.12.84 Patentblatt 84/49**

(45) Bekanntmachung des Hinweises auf die Patenterteilung:
**10.06.87 Patentblatt 87/24**

(84) Benannte Vertragsstaaten:
**AT BE CH DE FR GB IT LI LU NL SE**

(56) Entgegenhaltungen:
**DE-A-2 024 301**
**DE-A-2 425 939**
**DE-A-3 114 984**
**US-A-3 849 662**
**US-A-4 158 145**
**US-A-4 277 416**

**Die Akte enthält technische Angaben, die nach
dem Eingang der Anmeldung eingereicht wurden
und die nicht in dieser Patentschrift enthalten sind.**

(73) Patentinhaber: **KRAFTWERK UNION
AKTIENGESELLSCHAFT, Wiesenstrasse 35, D-4330
Mülheim (Ruhr) (DE)**

(72) Erfinder: **Goebel, Konrad, Dipl.- Ing., Stettiner
Strasse 5, D-8520 Erlangen (DE)**
Erfinder: **Müller, Rainer, Dr. Dipl.- Ing.,
Herdegenplatz 1, D-8520 Erlangen (DE)**
Erfinder: **Schiffers, Ulrich, Dr. Dipl.- Ing.,
Moritzbergstrasse 1, D-8501 Eckental (DE)**

(74) Vertreter: **Mehl, Ernst, Dipl.- Ing., Postfach 22 01
76, D-8000 München 22 (DE)**

EP 0 127 093 B1

LIBER, STOCKHOLM 1987

## Beschreibung

Die Erfindung bezieht sich auf ein Mittellastkraftwerk mit einer integrierten Kohlevergasungsanlage, mit einem an die Kohlevergasungsanlage angeschlossenen Gasturbinenkraftwerksteil, mit einem an die Rohgas-Wärmetauscheranlage der Kohlevergasungsanlage angeschlossenen Dampfkraftwerksteil und mit einer Methanolsyntheseanlage.

Die DE-OS 31 14 984 offenbart eine Kraftwerksanlage, bei der eine Gasturbine von einer Kohlevergasungsanlage mit Reingas versorgt wird. Die Gasturbine treibt einen elektrischen Generator an. Die Wärme der Abgase der Gasturbine werden bei dieser Kraftwerksanlage zur Dampferzeugung herangezogen. Mit dem Dampf wird eine Dampfturbine und ein weiterer elektrischer Generator angetrieben. Bei dieser Kraftwerksanlage ist auch vorgesehen, einen Teil des erzeugten Reingases einer Methanolsyntheseanlage zuzuführen und das erzeugte Methanol zu speichern. Die Leistung dieser Kraftwerksanlage läßt sich synchron mit der Leistung der Kohlevergasungsanlage regeln. Deren Leistung ist jedoch nur im Bereich zwischen etwa 75 % und 100 %, mit Einbußen an Wirtschaftlichkeit notfalls auch 55 % ihrer Nennleistung veränderbar. Es ist eine Eigenart dieser Kraftwerksanlage, daß Lastspitzen nur dadurch ausgefahren werden, daß das zuvor erzeugte Methanol zusätzlich zum Reingas in der Gasturbine mit verbrannt wird. Beim Abschalten des mit dem Kohlevergaser gekoppelten Kraftwerksteils muß auch die Methanolsyntheseanlage abgeschaltet werden, weil keine Möglichkeit besteht, die Wärme des Rohgases abzuführen.

Durch das europäische Patent 00 38 138 ist ein Mittellastkraftwerk bekannt, das zwei völlig unabhängige voneinander arbeitende Kraftwerksanlagen besitzt. Von diesen beiden Kraftwerksanlagen ist die erste Kraftwerksanlage, die eine Gasturbine mit einer am Abhitzekessel der Gasturbine angeschlossenen Dampfturbinenanlage umfaßt, an einer Kohlevergasungsanlage angeschlossen. Der Kohlevergasungsanlage ist außerdem eine Anlage zur Erzeugung von synthetischen Kraftstoffen nachgeschaltet. Die erste Kraftwerksanlage arbeitet im Grundlastbetrieb und ist nur soweit regelfähig, wie die vorgeschaltete Kohlevergasungsanlage. Diese läßt sich aber nur im Bereich von etwa 75 % bis 100 % ihrer Nennlast wirtschaftlich regeln. Ihr Lastverhalten ist maßgeblich durch dasjenige der Kohlevergasungsanlage einschließlich der dieser zugeordneten Luftzerlegungs anlage bestimmt. Die zweite, unabhängige Kraftwerksanlage gleicht im wesentlichen die Lastschwankungen der Stromproduktion aus. In ihr wird jedoch der wesentlich teurere zuvor erzeugte synthetische Kraftstoff verbrannt. Es ist eine Eigenart dieser Anlage, daß die überschüssige Reingasmenge bei plötzlichem Lastabwurf im Kraftwerksteil so lange abgefackelt werden muß, bis das Gleichgewicht zwischen der Reingaserzeugung und der Erzeugung von synthetischem Treibstoff bei der neuen niedrigeren Stromerzeugungsrate wieder hergestellt ist. Dieser Energieverlust kann beachtliche Ausmaße annehmen, weil das Herunterregeln einer größeren Kohlevergasungsanlage über eine Stunde dauern kann, während sich die Leistung einer Gasturbine in wenigen Minuten drosseln läßt. Beim Ausfahren von Lastspitzen, wie auch beim schnellen Hochfahren dieser Kraftwerksanlage muß der relativ teuere zuvor erzeugte synthetische Treibstoff in der zweiten unabhängigen Kraftwerksanlage verbrannt werden. Auch dies muß so lange geschehen, bis wieder ein Leistungsgewicht hergestellt ist.

Der Erfindung liegt die Aufgabe zugrunde, ein Mittellastkraftwerk zu entwickeln, das keine weitere unabhängige Kraftwerksanlage zum Auffangen stromseitiger Lastschwankungen benötigt. Auch Sollen bei diesem Mittellastkraftwerk Spitzenlastschwankungen ohne Verwendung eines teueren Zweitbrennstoffs ausführbar sein. Ferner soll jeder Brennstoffverlust bei plötzlicher Lastverringerung vermieden werden. Schließlich soll bei dem zu entwickelnden Mittellastkraftwerk der gesamte Wärmeinhalt der erzeugten Gase ausnutzbar sein.

Bei einem Mittellastkraftwerk der eingangs genannten Art ist daher erfindungsgemäß die Methanolsyntheseanlage aus parallel geschalteten Modulen zusammengesetzt und mit dem Gasturbinenkraftwerksteil über ein zentrales Reingasverteilungssystem verbunden, welches eine parallel zur Reingasversorgungsleitung geschaltete Reingas-Durchströmzwischenspeicheranlage umfaßt und gasseitig der Rohgas-Wärmetauscheranlage nachgeschaltet ist. Bei einem derart konzipierten Mittellastkraftwerk ist es möglich, bei Laständerung des Kraftwerksteils überschüssig aus der Kohleversorgungsanlage anfallende Gasmengen solange zwischenzuspeichern, bis das Gleichgewicht zwischen der Gaserzeugung und dem Gasbedarf in der Gesamtanlage wieder hergestellt ist. Das Gleichgewicht zwischen der Gasproduktion und dem Gasverbrauch kann bei abnehmender oder zunehmender Stromabgabe an das Netz durch Zuschalten oder Abschalten einzelner Module der Methanolsynthese stufenweise wieder hergestellt werden. Zwischenzeitig anfallende Mehr- oder Mindermengen können durch die an die Reingasversorgungsleitung angeschlossene Reingas-Durchströmzwischenspeicheranlage aufgenommen bzw. abgegeben werden.

In zweckmäßiger Ausgestaltung der Erfindung kann die Reingas-Durchströmzwischenspeicheranlage zur Konstanthaltung des Druckes in der

2

Reingasversorgungsleitung als Regel- und Zwischenspeicheranlage ausgebildet sein und einen Niederdruckspeicher und einen Hochdruckspeicher beinhalten, welche untereinadher über einen Druckerhöhungsverdichter verbunden sind. Ein solcher Reingas-Durchströmzwischenspeicher als funktioneller Bestandteil des zentralen Reingasverteilungssystems kann selbständig als Regel- und Zwischenspeicheranlage den Druck in der Reingasversorgungsleitung zwischen zwei Grenzwerten konstant halten. Hierdurch werden automatisch Gasmengendifferenzen zwischen der Erzeugung und dem Verbrauch ausgeglichen.

Die Ausnutzung der Wärme des Rohgases wird verbessert, wenn die Rohgaswärmetauscheranlage erfindungsgemäß drei Wärmetauscher umfaßt, von denen der erste und dritte der Dampferzeugung und der zweite der Vorwärmung des in der Brennkammer der Gasturbine des Gasturbinenkraftteils einströmenden Reingases dient. Diese Konzeption bringt den Vorteil, daß in dem ersten Wärmetauscher Hochdruckdamp erzeugt werden kann, der in den Hochdruckteil der Dampfturbine einspeisbar ist und zusätzlich im dritten Wärmetauscher Niederdruckdampf erzeugt werden kann, der in den Niederdruckteil der Dampfturbine einspeisbar ist, aber auch als Prozeßdampf verwendet werden kann. Außerdem wird dadurch die Voraussetzung für weitere Ausgestaltungen der Erfindung geschaffen.

So kann die Flexibilität des Mittellastkraftwerkes erhöht werden, wenn die Kapazität des ersten und dritten Wärmetauschers erfindungsgemäß derart bemessen ist, daß sie ausreicht, bei abgeschalteter Gasturbine und laufender Kohlevergasungsanlage die Dampfturbine zur Aufrechterhaltung der elektrischen Eigenversorgung der Kohleversorgungsanlage und der Methanolsyntheseanlage anzutreiben.

Außerdem kann so erfindungsgemäß, der dritte Wärmetauscher durch entsprechende Heizflächengestaltung derart ausgelegt sein, daß er die bei Teillast oder Stillstand der Gasturbine nun zusätzlich anfallende Rohgaswärme mit aufnehmen kann. Das hat zur Folge, daß das bei abgeschalteter Gasturbine mit höherer Temperatur in den dritten Wärmetauscher einströmende Rohgas dort auch eine größere Menge Dampf erzeugen kann. Dieser kann so zumindest teilweise die aus dem Abhitzekessel fehlende Dampfmenge substituieren.

Die Anpaßbarkeit des Mittellastkraftwerks an unterschiedliche Lastzustände wird insbesondere im unteren Lastbereich erhöht, wenn in zweckmäßiger Ausbildung der Erfindung das nicht vollständig umgesetzte Syntheseabgas in mindestens einem der Module der Methanolsyntheseanlage mit Hilfe eines Kreislaufverdichters über eine Wasserstoffanreicherungsstufe in den Synthesereaktor zurückführbar ist. Der Wasserstoff- und Kohlenmonoxydanteil des in diesen Modulen bei Schwachlast eingespeisten Reingases kann hier vollständig umgesetzt werden.

Die Methanolsyntheseanlage läßt sich vereinfachen, wenn in besonders zweckmäßiger Weiterbildung der Erfindung das im Synthesereaktor mindestens eines Moduls der Methanolsyntheseanlage nicht vollständig umgesetzte Syntheseabgas über eine Mischstrecke in die zum Gasturbinenkraftwerksteil führende Reingas-Versorgungsleitung einspeisbar ist. Mit dieser Ausbildung mindestens eines Moduls der Methanolsyntheseanlage werden gleich mehrere Vorteile erzielt. Dadurch, daß dieses Modul als Durchlaufsynthesemodul, d.h. ohne Kreislaufverdichter und ohne Wasserstoffanreicherungsstufe ausgebildet ist, sind dessen Kapital- und Energiekosten geringer als bei Modulen, in denen das Synthesegas rezirkuliert. Folglich ist das Methanol hier preisgünstiger herstellbar. Darüber hinaus haben die Synthesegase dieses Moduls der Methanolsyntheseanlage einen ausreichend hohen Heizwert um über eine Mischstrecke in die zum Gasturbinenkraftwerksteil führende Reingasversorgungsleitung einspeisbar zu sein. Dem kommt auch zugute, daß dieses Modul bei allen Betriebszuständen des Mittellastkraftwerks in Betrieb bleibt, so daß die in die Reingasversorgungsleitung eingespeiste Gasmenge annähernd konstant ist und einen entsprechenden Anteil an Reingas substituiert. Diese Ausbildung und Betriebsweise dieses Moduls bildet zugleich auch die Voraussetzung für eine andere Ausgestaltung der Erfindung.

So kann in zweckmäßiger Ausgestaltung der Erfindung das nicht vollständig umgesetzte Syntheseabgas mindestens eines Moduls der Methynolsyntheseanlage zur beschleunigten Inbetriebsetzung des Synthesereaktors eines der übrigen Module in die in deren Synthesereaktor zurückführende Rezirkulationsleitung einspeisbar sein. Hierdurch wird erreicht, daß bei schnell steigendem Reingasangebot, wie beispielsweise beim Abfahren der Gasturbine, Lastabwurf od.dgl. weitere Module der Methanolsyntheseanlage durch Einspeisen des nicht vollständig umgesetzten heißen Syntheseabgabes eines im Betrieb befindlichen Moduls beschleunigt aufgeheizt und somit wesentlich schneller in Betrieb genommen werden können. Indirekt werden hierdurch die Anforderungen an die Speicherkapazität der Reingas-Durchströmzwischenspeicheranlage verringert.

Weitere Einzelheiten der Erfindung werden anhand eines Ausführungsbeispiels erläutert. Es zeigt

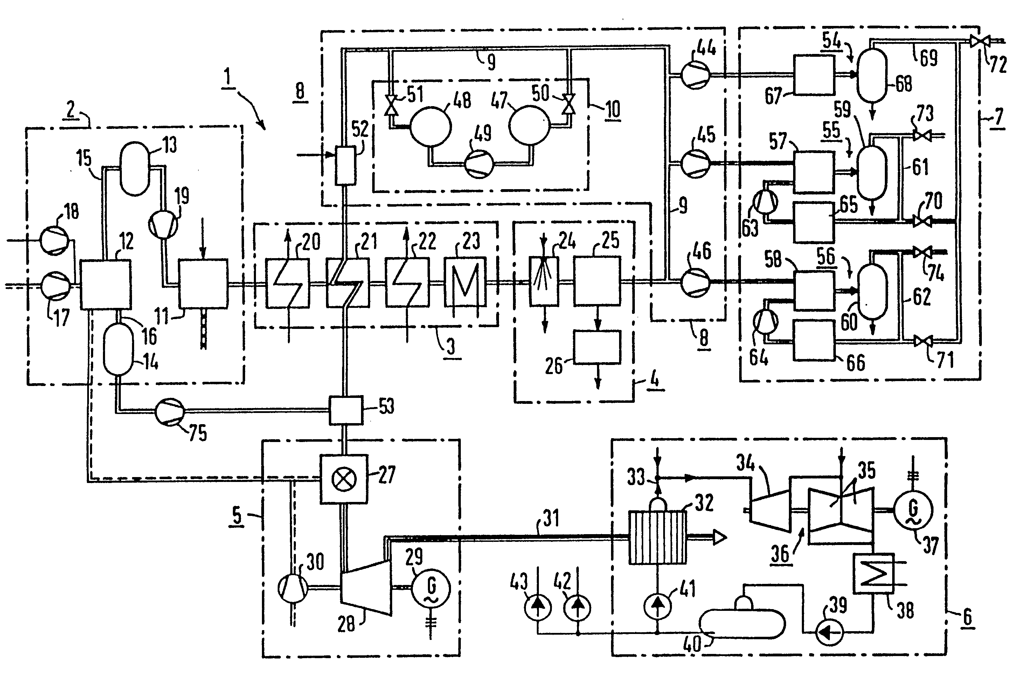

Die Figur eine schematische Darstellung des erfindungsgemäßen Mittellastkraftwerkes.

Die wesentlichsten Baugruppen des im Ausführungsbeispiel dargestellten erfindungsgemäßen Mittellastkraftwerkes 1 sind eine Kohlevergasungsanlage 2, eine Rohgas- Wärmetauscheranlage 3, eine Gasreinigungsanlage 4, ein aus einem Gasturbinenkraftwerksteil 5 und einem Dampfkraftwerksteil 6 bestehendes Kombikraftwerk, eine Methanolsyntheseanlage 7 und ein zentrales Reingasverteilungssystem 8 mit einer parallel zur Reingasversorgungsleitung 9 geschalteten Reingasdurchströmungszwischenspeicheranlage 10. Die Kohlevergasungsanlage 2 beinhaltet einen Kohlevergaser 11, eine Luftzerlegungsanlage 12, je einen Pufferspeicher 13, 14 in der Sauerstoffleitung 15 und der Stickstoffleitung 16 der Luftzerlegungsanlage 12, zwei der Luftzerlegungsanlage vorgeschaltete Zusatzluftverdichter 17, 18 und einen in der Sauerstoffleitung 15 zum Kohlevergaser 11 angeordneten weiteren Gasverdichter 19. Die im Gasstrom des Kohlevergasers angeordnete Rohgaswärmetauschanlage 3 beinhaltet einen ersten der Dampferzeugung dienenden Wärmetauscher 20, einen zweiten der Reingasvorwärmung dienenden Rohgas-Reingas-Wärmetauscher 21 und einen dritten, ebenfalls der Dampferzeugung dienenden Wärmetauscher 22. Schließlich ist in der Rohgas-Wärmetauschanlage 3 noch ein Regelkühler 23 vorgesehen. Die der Rohgas-Wärmetauscheranlage 3 nachgeschaltete Gasreinigungsanlage 4 beinhaltet einen Rohgaswäscher 24, eine Schwefelwasserstoffabsorptionsanlage 25 und eine Schwefelgewinnungsanlage 26.

An der den Rohgas-Reingas-Wärmetauscher 21 verlassenden Reingasversorgungsleitung ist der Gasturbinenkraftwerksteil 5 angeschlossen. Im Ausführungsbeispiel umfaßt es nur eine Brennkammer 27, eine Gasturbine 28 und je einen von der Gasturbine angetriebenen Generator 29 und Luftverdichter 30. An der die Gasturbine verlassenden Abgasleitung 31 ist ein Abhitzekessel 32 vorgesehen, an dessen Dampfleitung 33 die aus einem Hochdruckteil 34 und einem Niederdruckteil 35 bestehende Dampfturbine 36 des Dampfkraftwerksteils 6 angeschlossen ist. Die Dampfturbine treibt einen Generator 37 an. Dem Niederdruckteil 35 der Dampfturbine 36 sind ein Kondensator 38, eine Kondensatpumpe 39, ein Speisewasserbehälter 40 sowie mehrere Speisewasserpumpen 41, 42, 43 nachgeschaltet.

Das zentrale Reingasverteilungssystem 8 umfaßt außer der Reingasversorgungsleitung 9 und der parallel zur Reingasversorgungsleitung geschalteten Reingasdurchström-Zwischenspeicheranlage 10 auch die die Methanolsyntheseanlage 7 versorgenden Reingasverdichter 44, 45, 46. Die Reingasdurchström-Zwischenspeicheranlage beinhaltet einen Niederdruckspeicher 47 und einen Hochdruckspeicher 48 und einen zwischengeschalteten Reingasverdichter 49. Dabei ist der Niederdruckspeicher 47 über ein Ladeventil 50 und der Hochdruckspeicher 48 über ein Entladeventil 51 mit der Reingasversorgungsleitung 9 verbunden. Das Entladeventil 51 wird in hier nicht weiter dargestellter Weise über Druckfühler aufgesteuert, wenn der Druck in der Reingasversorgungsleitung 9 unter einen voreingestellten Wert sinkt. Das Ladeventil 50 wird aufgesteuert, wenn der Druck in der Reingasversorgungsleitung 9 über einen voreingestellten Wert steigt. In der zum Rohgas-Reingasvorwärmer 21 führenden Reingasversorgungsleitung 9 ist eine Mischstrecke 52 für die Zumischung von Synthesegas aus der Methanolsyntheseanlage 7 vorgesehen. Unmittelbar vor der Brennkammer 27 der Gasturbine 28 ist ferner eine Mischkammer 53 für die Zumischung von Stickstoff zum Reingas vorgesehen.

Die Methanolsyntheseanlage 7 besteht im Ausführungsbeispiel aus drei parallelgeschalteten Modulen 54, 55, 56, von denen zwei Module 55, 56 aus einem Synthesereaktor 57, 58, einem Methanolabscheider 59, 60, einer die Syntheseabgase des Methanolabscheiders in den Synthesereaktor zurückführende Rezirkulationsleitung 61, 62 mit einem Kreislaufverdichter 63, 64 und einer Wasserstoffanreicherungsstufe 65, 66 bestehen. Ein weiteres Modul 54 der Methanolsyntheseanlage 7 ist lediglich mit einem Synthesereaktor 67 und einem diesem nachgeschalteten Methanolabscheider 68 ausgerüstet. Dessen Syntheseabgasleitung 69 ist über Ventile 70, 71, 72 mit den Rezirkulationsleitungen 61, 62 der übrigen Module 55, 56 und mit der Mischstrecke 52 in der Reingasversorgungsleitung 9 verbunden.

Bei Nennlastbetrieb wird die Luftzerlegungsanlage 12 sowohl durch den von der Gasturbine 28 angetriebenen Luftverdichter 30 als auch über mindestens einen der Zusatzluftverdichter 17, 18 der Luftzerlegungsanlage mit Druckluft versorgt. Der Sauerstoff der Luftzerlegungsanlage wird über den Pufferspeicher 13 und den Gasverdichter 19 in den Kohlevergaser 11 eingeblasen. Im Kohlevergaser wird die Kohle mit dem Sauerstoff und eingeleitetem Prozeßdampf zu Rohgas vergast. Das 800 bis 1.600° heiße Rohgas gibt zunächst einen Teil seiner Wärme im ersten Wärmetauscher 20 der Rohgas-Wärmetauscheranlage 3 ab, in welchem Hochdruckdampf zur Einspeisung in den Hochdruckteil 34 der Dampfturbine 36 erzeugt wird. Im zweiten Wärmetauscher 21 der Rohgas-Wärmetauscheranlage wird mit der Abwärme des Rohgases das zur Brennkammer 27 der Gasturbine 28 strömende Reingas vorgewärmt. Weitere Wärme wird dem Rohgas im dritten Wärmetauscher 22, in dem Niederdruckdampf erzeugt wird, entzogen. Dieser Niederdruckdampf wird bei Nennlastbetrieb teilweise in den Niederdruckteil 35 der Dampfturbine 36 eingespeist und teilweise als Prozeßdampf verwendet und zum Beispiel in den Kohlevergaser 11 eingeleitet. In dem sich dem dritten Wärmetauscher 22 der Rohgas-Wärmetauscheranlage 3 anschließenden Regelkühler 23 wird die Rohgastemperatur vor Eintritt in die nachgeschaltete Gasreinigungsanlage 4 auf eine vorgegebene Temperatur geregelt.

In der Gasreinigungsanlage 4 wird das Rohgas zunächst im Rohgaswäscher 24 von Staubteilchen und in der nachfolgenden Schwefelwasserstoffabsorptionsanlage 25 von Schwefelwasserstoff gerinigt. Das Schwefelwasserstoffhaltige Abgas der Schwefelwasserstoffabsorptionsanlage 25 wird in der Schwefelgewinnungsanlage 26 zu Schwefel umgewandelt. Das die Gasreinigungsanlage 4 verlassende Reingas wird über die Reingasversorgungsleitung 9 der Reingasdurchströmzwischenspeicheranlage 10 sowie den anderen Gasverbrauchern zugeleitet. Über die Reingasverdichter 44, 45, 46 wird das Reingas bei den in Betrieb befindlichen Moduln der Methanolsyntheseanlage 7 auf Synthesedruck verdichtet und in den jeweiligen Methanolsynthesereaktor eingespeist. Bei Nennlastbetrieb ist bevorzugt nur das Modul 54, das im Durchlaufsynthesebetrieb läuft, in Betrieb. Sein den Methanolsynthesereaktor 67 verlassendes Synthesegas wird im nachgeschalteten Methanolabscheider 68 vom Methanol befreit. Das aus dem Methanolabscheider 68 ausströmende Syntheseabgas ist nur teilweise umgesetzt und hat daher noch einen Heizwert, der sich nicht allzusehr vom Heizwert des Reingases unterscheidet. Das anfallende Syntheseabgas, kann über die Mischstrecke 52 in die zur Brennkammer 27 der Gasturbine führende Reingasleitung eingespeist werden. Es substituiert dort einen Teil des Reingases.

Die beiden anderen mit je einer Rezirkulationsleitung 61, 62 versehenen Module 55, 56 werden dann zugeschaltet, wenn ein Überschuß an Reingas verfügbar ist, weil beispielsweise die Leistung der Gasturbine 28 zurückgenommen wurde und diese Reingasmenge nicht durch Hochfahren der bereits im Betrieb befindlichen Module 54 aufgefangen werden kann. In ihnen wird das Syntheseabgas über die Rezirkulationsleitung 61, 62 und eine Wasserstoffanreicherungsstufe 65, 66 in den Methanolsynthesereaktor 57, 58 zurückgeführt. In der Wasserstoffanreicherungsstufe wird das für die Methanolsynthese erforderliche stöchiometrische Verhältnis von $H_2$ und $CO = 2$ durch Wasserstoffzugabe wieder hergestellt. Die Wasserstoffanreicherungsstufen könnten auch in den Reingasleitungen zu den Synthesereaktoren statt in den Rezirkulationsleitungen eingebaut sein. Durch Rezirkulation des Syntheseabgases können die Wasserstoff- und Kohlenmonoxidanteile des Syntheseabgases nahezu vollständig umgesetzt werden. Zur Aufrechterhaltung einer konstanten Menge an inerten Gasen im rezirkulierenden Syntheseabgas werden geringe Mengen des Syntheseabgases als Restgas über die Ventile 73, 74 abgelassen und in einem hier nicht weiter dargestellten Dampferzeuger verbrannt. Dessen Dampf kann als Prozeßdampf oder auch als Dampf zum Betrieb einer separaten Dampfturbine benutzt werden.

Alternativ kann dieses Restgas auch in einer separaten Gasturbine verbrannt werden. Mit dieser Dampfturbine bzw. Gasturbine kann über einen Generator elektrische Energie zur Deckung des Eigenbedarfs des Mittellastkraftwerkes 1 erzeugt werden.

Die Gasturbine 28 treibt den Generator 29 und den Luftverdichter 30 an. Diese versorgt sowohl die Brennkammer 27 der Gasturbine als auch die Luftzerlegungsanlage 12 mit Druckluft. Weil die Leistung des Luftverdichters 30 an die Luftmenge angepaßt ist, den die Gasturbine 28 bei Vollast benötigt, muß zur Deckung des Gesamtsauerstoffbedarfes der Kohlevergasungsanlage 2 bei Vollast des Gasturbinenkraftwerksteils 5 und bei Betrieb des Moduls 52 der Methanolsyntheseanlage ein regelbarer Zusatzluftverdichter 17 in Betrieb sein. Dieser Zusatzluftverdichter 17 so wie weitere parallelgeschaltete Zusatzluftverdichter 18 liefern die bei Stillstand der Gasturbine 28 von der Luftzerlegungsanlage 12 für den nachgeschalteten Kohlevergaser 11 benötigte Luftmenge um die Methanolsyntheseanlage 7 weiter zu betreiben.

Zur Minderung der $NO_X$-Bildung bei der Verbrennung des Reingases wird dem Reingas Stickstoff aus der Luftzerlegungsanlage 12 vor Eintritt in die Brennkammer 27 mit Hilfe eines Verdichters 75 zugemischt. Hierdurch wird die Temperatur in der Flamme und damit die $NO_X$-Erzeugung vermindert. Die zugemischte Stickstoffmenge ist an die Aufnahmefähigkeit der Gasturbine im jeweiligen Betriebszustand angepaßt. Überschüssiger, von der Gasturbine nicht aufnehmbarer Stickstoff, kann in den Pufferspeicher 14 aufgefangen werden. Wenn der Gasturbine bei verringerter Last weniger Reingas zugeleitet wird, so kann, in gewissen Grenzen, mehr Stickstoff zugemischt werden.

Die heißen Abgase der Gasturbine 28 werden über die Abgasleitung 31 in den Abhitzekessel 32 geleitet. Ihre Abwärme wird zur Dampferzeugung verwendet. Der im Abhitzekessel erzeugte Dampf, sowie zusätzlich in der Rohgas-Wärmetauscheranlage 3 erzeugter Dampf werden der Dampfturbine 36 zugeleitet. Der den Niederdruckteil 35 der Dampfturbine verlassende Dampf wird im Kondensator 38 kondensiert. Das Kondensat wird in den Speisewasserbehälter 40 gepumpt, von wo es über die Speisewasserpumpen 41, 42, 43 in den Abhitzekessel 32 und die übrigen Wärmetauscher 20, 22 zurückbefördert werden kann.

Wird die Antriebsleistung der Gasturbine 28 zurückgenommen, so vermindert sich auch der Reingasdurchsatz durch den Reingas-Rohgas-Wärmetauscher 21. Dies führt zu einer höheren Eintrittstemperatur des Rohgases in den dritten Wärmetauscher 22. Dieser ist aber so ausgelegt und bemessen, daß er selbst bei vollständiger Abschaltung der Gasturbine und fehlender Rohgaskühlung im Rohgas-Reingas-Wärmetauscher 21 das vergrößerte Wärmeangebot des Rohgases voll aufnehmen kann. In ihm entsteht durch Anpassung der Speisewassereinspeisung eine entsprechend größere Dampfmenge, die dem Niederdruckteil 35 der Dampfturbine 36 zuleitbar ist und das geringere Dampfangebot des Abhitzekessels 32 der Gasturbine 28 teilweise kompensiert.

Infolge der Rücknahme der Gasturbinenleistung steht dem gleichbleibenden Gasangebot der Kohlevergasungsanlage 11 eine verminderte Gasabnahme gegenüber. Dies führt zu einer Erhöhung des Druckes in der Reingasversorgungsleitung 9 über einen voreingestellten Solldruck hinaus und somit zu einem Ansprechen des Ladeventils 50 der Reingasdurchström-Zwischenspeicheranlage 10. Über das Ladeventil 50 wird zunächst der Niederdruckspeicher 47 und sodann über den Reingasverdichter 49 auch der Hochdruckspeicher 48 geladen. Zugleich wird die Leistung der im Betrieb befindlichen Module 54 der Methanolsyntheseanlage 7 gesteigert. Wenn das nicht ausreicht um das Gleichgewicht zwischen Gasangebot und Gasabnahme zu erreichen, werden weitere Module 55, 56 der Methanolsyntheseanlage 7 in Betrieb genommen. Hierzu wird über eines der in die Rezirkulationsleitung 61, 62 eines der in Betrieb zu setzenden Module mündenden Ventile 70, 71 heißes Syntheseabgas eines im Betrieb befindlichen Moduls 54 eingeleitet und deren Synthesereaktor 57, 58 über die Wasserstoffanreicherungsstufe 65, 66 und den Kreislaufverdichter 63, 64 mit heißem Syntheseabgas aufgeheizt. Dieses Aufheizen erfolgt zusätzlich zum Aufheizen über den einzelnen Modulen zugeordnete, in der Figur nicht weiter dargestellte, Wärmetauscher. Durch diese zweifache Aufheizung können diese weiteren Module 55, 56 beschleunigt in Betrieb genommen werden. Dabei werden so viele Module nacheinander zugeschaltet, bis annähernd wieder ein Gleichgewicht zwischen dem Gasangebot und der Gasabnahme besteht.

Bei vollständig abgeschalteter Gasturbine 28 führt das dazu, daß alle Module der Methanolsyntheseanlage eingeschaltet sind und zusammen die von der Kohlevergasungsanlage 2 gelieferte Reingasmenge vollständig aufnehmen. Das kann je nach Dimensionierung der Methanolsyntheseanlage 7 die Reingasmenge sein, die der Kohlevergaser 11 bei Nennlast oder etwas reduzierter Last liefert. Bei abgeschalteter Gasturbine kann jedoch die Luftzerlegungsanlage 12 nicht über den Luftverdichter 30 der Gasturbine 28 mit Druckluft versorgt werden, sondern muß über die der Luftzerlegungsanlage zugeordneten Zusatzluftverdichter 17, 18 versorgt werden. Als Zusatzluftverdichter kann ein einzelner regelbarer oder auch mehrere parallelgeschaltete Zusatzluftverdichter 17, 18 verwendet werden. Die Antriebsenergie für die Zusatzluftverdichter wird dem ersten und dritten Wärmetauscher der Wärmetauscheranlage 3 entnommen. Deren Dampfleistung reicht aus, um die Dampfturbine 36 anzutreiben und elektrische Energie für den Eigenbedarf der Kohlevergasungsanlage 2 sowie der Methanolsyntheseanlage 7 mit den ihr zugeordneten Verdichtern 17, 18, 19, 44, 45, 46, 63, 64 zu erzeugen. Bei vollständig abgeschalteter Gasturbine wird das gesamte Syntheseabgas des Durchlaufsynthesemoduls 54 in die Rezirkulationsleitungen 61, 62 der übrigen Module 55, 56 mit eingespeist.

Wird die Gasturbine 28 bei steigendem Leistungsbedarf wieder in Betrieb genommen, so steht dem unveränderten Reingasangebot zunächst ein vergrößerter Verbrauch gegenüber. Das führt zu einem Absinken des Druckes in der Reingasversorgungsleitung 9 unter den Solldruck. Dies wiederum hat zur Folge, daß das Entladeventil 51 der Durchströmungs-Zwischenspeicheranlage 10 anspricht. Nun strömt so lange Reingas aus dem Hochdruckspeicher 48 in die Reingasversorgungsleitung 9 ein bis der Solldruck wieder erreicht ist. Gleichzeitig wird das Gleichgewicht zwischen Reingasangebot und Reingasbedarf durch Abschalten bzw. Herunterregeln einzelner Module 55, 56 der Methanolsyntheseanlage 7 wieder hergestellt. Geringere Differenzbeträge zwischen Reingasangebot und Reingasnachfrage werden dabei laufend durch die Reingasdurchström-Zwischenspeicheranlage 10 ausgeglichen. Mit dem Wiederanlaufen der Gasturbine 28 steht auch wieder Druckluft aus dem Luftverdichter 30 der Gasturbine 28 zur Verfügung, die, so lange die Gasturbine nicht mit Vollast betrieben wird, in der der Gasturbine zugeordneten Brennkammer 27 nicht vollständig benötigt wird. Diese überschüssige Luftmenge kann der Luftzerlegungsanlage 12 zugeleitet werden, so daß die Leistung der Zusatzverdichter 17, 18 zurückgenommen werden kann. Gleichzeitig steht wegen des nun wieder in Betrieb befindlichen Rohgas-Reingas-Wärmetauschers 21 dem verringerten Dampfangebot aus dem dritten Wärmetauscher 22 wieder ein zusätzliches Dampfangebot aus dem Abhitzekessel 32 der Gasturbine 26 gegenüber. Dabei vergrößert sich das Gesamtdampfangebot, so daß sich auch die Leistung der Dampfturbine 36 vergrössert und aus deren Generator mehr elektrische Leistung ans Netz abgegeben werden kann.

Beim Ausführungsbeispiel ist die Kohlevergasungsanlage 2 mit einem Druck betrieben worden, der dem Druck entspricht, den die Brennkammer 27 der Gasturbine 28 benötigt. Dieser Druck liegt bedeutend niedriger als der Druck den die Methanolsynthesereaktoren 57, 58, 67 zum Betrieb benötigen. Daher sind zu deren Anschluß Reingasverdichter 44, 45, 46 erforderlich. Diese Reingasverdichter kann man einsparen, wenn man den Druck im Kohlevergaser entsprechend erhöht. In diesem Fall muß aber eine Entspannungsturbine in der Reingasleitung 9 vor der Brennkammer 27 der Gasturbine vorgesehen werden. In dieser Entspannungsturbine kann ein Teil der Energie zurückgewonnen werden, die die den Kohlevergaser vorgeschalteten Verdichter verbrauchen.

**Bezugszeichenliste**

| | |
|---|---|
| Mittellastkraftwerk | 1 |
| Kohlevergasungsanlage | 2 |
| Rohgas-Wärmetauscheranlage | 3 |
| Gasreinigungsanlage | 4 |
| Gasturbinenkraftwerksteil | 5 |
| Dampfkraftwerksteil | 6 |
| Methanolsyntheseanlage | 7 |
| Reingasverteilungssystem | 8 |
| Reingasversorgungsleitung | 9 |
| Reingas-Durchstromzwischen-speicheranlage | 10 |
| Kohlevergaser | 11 |
| Luftzerlegungsanlage | 12 |
| Pufferspeicher | 13, 14 |
| Sauerstoffleitung | 15 |
| Stickstoffleitung | 16 |
| Zusatzluftverdichter, | 17, 18 |
| Gasverdichter | 19 |
| Wärmetauscher | 20, 22 |
| Rohgas-Reingaswärme-tauscher | 21 |
| Regelkühler | 23 |
| Rohgaswäscher | 24 |
| Schwefelwasserstoff-absorptionsanlage | 25 |
| Schwefelgewinnungsanlage | 26 |
| Brennkammer | 27 |
| Gasturbine | 28 |
| Generator | 29 |
| Luftverdichter | 30 |
| Abgasleitung | 31 |
| Abhitzekessel | 32 |
| Dampfleitung | 33 |
| Hochdruckteil | 34 |
| Niederdruckteil | 35 |
| Dampfturbine | 36 |
| Generator | 37 |
| Kondensator | 38 |
| Kondensatpumpe | 39 |
| Speisewasserbehälter | 40 |
| Speisewasserpumpe | 41, 42, 43 |
| Reingasverdichter | 44, 45, 46 |
| Niederdruckspeicher | 47 |
| Hochdruckspeicher | 48 |
| Reingasverdichter | 49 |
| Ladeventil | 50 |
| Entladeventil | 51 |
| Mischstrecke | 52 |
| Mischkammer | 53 |
| Modul | 54, 55, 56 |
| Synthesereaktor | 57, 58 |
| Methanolabscheider | 59, 60 |
| Rezirkulationsleitung | 61, 62 |
| Kreislaufverdichter | 63, 64 |
| Wasserstoff-anreicherungsstufe | 65, 66 |
| Synthesereaktor | 67 |
| Methanolabscheider | 68 |
| Syntheseabgasleitung | 69 |
| Ventil | 70, 71, 72 |
| Ventil | 73, 74 |
| Verdichter | 75 |

**Patentansprüche**

1. Mittellastkraftwerk (1) mit einer integrierten Kohlevergasungsanlage (2), mit einem an die Kohlevergasungsanlage angeschlossenen Gasturbinenkraftwerksteil (5), mit einem an die Rohgaswärmetauscheranlage (3) der Kohlevergasungsanlage angeschlossenen Dampfkraftwerksteil (6) und mit einer Methanolsyntheseanlage (7), dadurch gekennzeichnet, daß die Methanolsyntheseanlage (7) aus parallelgeschalteten Modulen (54, 55, 56) zusammengesetzt ist und mit dem Gasturbinenkraftwerksteil über ein zentrales Reingasverteilungssystem (8) verbunden ist, welches eine parallel zur Reingasversorgungsleitung (9) geschaltete Reingasdurchström-Zwischenspeicheranlage (10) umfaßt und gasseitig der Rohgas-Wärmetauscheranlage (3) nachgeschaltet ist.

2. Mittellastkraftwerk nach Anspruch 1, dadurch gekennzeichnet, daß die Reingas-Durch-Strömzwischenspeicheranlage (10) zur Konstanthaltung des Druckes in der Reingasversorgungsleitung (9) als Regel- und Zwischenspeicheranlage ausgebildet ist und einen Niederdruckspeicher (47) und einen Hochdruckspeicher (48) beinhaltet, welche untereinander über einen Druckerhöhungsverdichter (49) verbunden sind.

3. Mittellastkraftwerk nach Anspruch 1, dadurch gekennzeichnet, daß die Rohgas-Wärmetauscheranlage (3) drei Wärmetauscher (20, 21, 22) umfaßt, von denen der erste (20) und dritte (22) der Dampferzeugung und der zweite der Vorwärmung des in die Brennkammer (27) der Gasturbine (28) des Gasturbinenkraftwerksteiles (5) einströmenden Reingases dient.

4. Mittellastkraftwerk nach Anspruch 3, dadurch gekennzeichnet, daß die Kapazität des ersten und dritten Wärmetauschers (20, 22) derart bemessen ist, daß sie ausreicht, bei abgeschalteten Gasturbinen (28) und laufender Kohlevergasungsanlage (2) die Dampfturbine (36) zur Aufrechterhaltung der elektrischen Eigenversorgung der Kohlevergasungsanlage (2) und der Methanolsyntheseanlage (7) anzutreiben.

5. Mittellastkraftwerk nach Anspruch 3 oder 4, dadurch gekennzeichnet, daß der dritte Wärmetauscher (22) durch entsprechende Heizflächengestaltung derart ausgelegt ist, daß er die bei Teillast oder Stillstand der Gasturbine (28) zusätzlich anfallende Rohgaswärme mit aufnehmen kann.

6. Mittellastkraftwerk nach Anspruch 1, dadurch gekennzeichnet, daß das nicht vollständig umgesetzte Syntheseabgas in mindestens einem der Module (55, 56) der Methanolsyntheseanlage (7) mit Hilfe eines Kreislaufverdichters (63, 64) über eine Wasserstoffanreicherungsstufe (65, 66) in den Synthesereaktor (57, 58) zurückführbar ist.

7. Mittellastkraftwerk nach Anspruch 1, dadurch gekennzeichnet, daß das im Synthesereaktor (67) mindestens eines Moduls (57) der Methanolsyntheseanlage (7) nicht vollständig umgesetzte Syntheseabgas über eine Mischstrecke (52) in die zum Gasturbinenkraftwerksteil (5) führende Reingasversorgungsleitung (9) einspeisbar ist.

8. Mittellastkraftwerk nach Anspruch 6 oder 7, dadurch gekennzeichnet, daß das nicht vollständig umgesetzte Syntheseabgas mindestens eines Modzuls (54) der Methanolsyntheseanlage (7) zur beschleunigten Inbetriebsetzung des Synthesereaktors eines der übrigen Modzule (55, 56) in die in deren Synthesereaktor (57, 58) zurückführende Rezirkulationsleitung (61, 62) einspeisbar ist.

9. Mittellastkraftwerk nach Anspruch 1 oder 6, dadurch gekennzeichnet, daß das aus den Rezirkulationsleitungen (61, 62) abgezogene Restgas der einzelnen Module (55, 56) der Methanolsyntheseanlage (7) in einem separaten Dampferzeuger verbrannt wird und der erzeugte Dampf in den Dampfkraftwerksteil (6) einspeisbar ist.

10. Mittellastkraftwerk nach Anspruch 1 oder 6, dadurch gekennzeichnet, daß das durch Rezirkulation weitgehend umgesetzte Restgas der einzelnen Module (55, 56) der Methanolsyntheseanlage (7) in einer separaten Gasturbine zur Deckung des eigenbedarfs an elektrischer Energie verbrannt wird.

11. Mittellastkraftwerk nach Anspruch 3, dadurch gekennzeichnet, daß ein wassergekühlter Regelkühler (23) zur Konstanthaltung der Rohgasaustrittstemperatur der Rohgas-Wärmetauscheranlage (3) verwendet ist.

12. Mittellastkraftwerk nach Anspruch 1, dadurch gekennzeichnet, daß dem Kohlevergaser (11) mindestens ein zusätzlicher Luftverdichter (17, 18) zugeordnet ist, der parallel zum Luftverdichter (30) des Gasturbinenkraftwerksteils (5) geschaltet ist und durch den die Versorgung der dem Kohlevergaser vorgeschalteten Luftzerlegungsanlage (12) mit Luft ergänzbar ist.

13. Mittellastkraftwerk nach anspruch 12, dadurch gekennzeichnet, daß der zusätzliche Luftverdichter (17, 18) bei abgeschaltetem Gasturbinenkraftwerksteil (5) die Versorgung der Kohlevergasungsanlage (2) für den Betrieb der Methanolsyntheseanlage (7) übernimmt.

14. Mittellastkraftwerk nach Anspruch 1, dadurch gekennzeichnet, daß ein Sauerstoffpufferspeicher (13) zwischen dem Kohlevergaser (11) und der dem Kohlevergaser vorgeschalteten Luftzerlegungsanlage (12) vorgesehen ist.

15. Mittellastkraftwerk nach Anspruch 1, dadurch gekennzeichnet, daß die Stickstoffleitung der dem Kohlevergaser (11) vorgeschalteten Luftzerlegungsanlage (12) über einen Pufferspeicher (14) mit der zum Brenner (27) des Gasturbinenkraftwerksteil (5) führenden Reingasleitung (9) verbunden ist.

## Claims

1. Intermediate power plant (1) with an integrated coal gasification installation (2), with a gas turbine power plant section (5) connected to the coal gasification installation, with a steam power plant section (6) connected to the crude gas heat exchanger installation (3) of the coal gasification installation and with a methanol synthesis installation (7), characterised in that the methanol synthesis installation (7) is composed of modules (54, 55, 56) connected in parallel and is connected with the gas turbine power plant section via a central pure gas distribution system (8) which comprises a pure gas through-flow intermediate storage installation (10) connected in parallel to the pure gas supply line (9) and which is connected after the crude gas heat exchanger installation (3) on the gas side.

2. Intermediate power plant according to claim 1, characterised in that the pure gas through-flow intermediate storage installation (10) for keeping the pressure in the pure gas supply line (9) constant is developed as a regulating and intermediate storage installation and contains a low pressure store (47) and a high pressure store (48) which are interconnected via a pressure increasing compressor (49).

3. Intermediate power plant according to claim 1, characterised in that the crude gas heat exchanger installation (3) comprises three heat exchangers (20, 21, 22), of which the first (20) and the third (22) serve to generate steam and the second serves to preheat the pure gas flowing into the combustion chamber (27) of the gas turbine (28) of the gas turbine power plant section (5).

4. Intermediate power plant according to claim 3, characterised in that the capacity of the first and third heat exchanger (20, 22) is rated such that it is sufficient, with the gas turbines (28) shut off and the coal gasification installation (2) running, to drive the steam turbine (36) in order to maintain the electrical self-sufficiency of the coal gasification installation (2) and of the methanol synthesis installation (7).

5. Intermediate power plant according to claim 3 or 4, characterised in that the third heat exchanger (22) is designed, through corresponding shaping of the heating surfaces, in such a way that it can also receive the crude gas heat yielded additionally when the gas turbine (28) is partially loaded or at a standstill.

6. Intermediate power plant according to claim 1, characterised in that the synthesis waste gas, which is not completely converted, in at least one of the modules (55, 56) of the methanol synthesis installation (7) can be returned to the synthesis reactor (57, 58) with the aid of a circulatory compressor (63, 64) via a hydrogen enrichment stage (65, 66).

7. Intermediate power plant according to claim 1, characterised in that the synthesis waste gas which is not completely converted in the synthesis reactor (67) of at least one module (57) of the methanol synthesis installation (7) can be fed via a mixing stretch (52) into the pure gas supply line (9) leading to the gas turbine power plant section (5).

8. Intermediate power plant according to claim 6 or 7, characterised in that the synthesis waste gas, which is not completely converted, of at least one module (54) of the methanol synthesis installation (7) can, in order to accelerate actuation of the synthesis reactor of one of the other modules (55, 56), be fed into the recirculation line (61, 62) leading back into their synthesis reactor (57, 58).

9. Intermediate power plant according to claim 1 or 6, characterised in that the residual gas of the individual modules (55, 56) of the methanol synthesis installation (7), drawn off from the recirculation lines (61, 62) is burnt in a separate steam generator and the steam generated can be fed into the steam power plant section (6).

10. Intermediate power plant according to claim 1 or 6, characterised in that the residual gas, for the most part converted through recirculation, of the individual modules (55, 56) of the methanol synthesis installation (7) is burnt in a separate gas turbine in order to cover the internal consumption of electrical energy.

11. Intermediate power plant according to claim 3, characterised in that a water-cooled regulating cooler (23) is used to keep the crude gas outlet temperature of the crude gas heat exchanger installation (3) constant.

12. Intermediate power plant according to claim 1, characterised in that coordinated with the coal gasifier (11) there is at least one additional air compressor (17, 18) which is connected in parallel to the air compressor (30) of the gas turbine power plant section (5) and through which the supply of the air separation installation (12) connected upstream of the coal gasifier can be supplemented with air.

13. Intermediate power plant according to claim 12, characterised in that the additional air compressor (17, 18) takes over the supply of the coal gasification installation (2) for the operation of the methanol synthesis installation (7) when the gas turbine power plant section (5) is shut down.

14. Intermediate power plant according to claim 1, characterised in that an oxygen buffer store (13) is provided between the coal gasifier (11) and the air separation installation (12) connected upstream of the coal gasifier.

15. Intermediate power plant according to claim 1, characterised in that the nitrogen line of the air separation installation (12) connected upstream of the coal gasifier (11) is connected via a buffer store (14) with the pure gas line (9) leading to the burner (27) of the gas turbine power plant section (5).

## Revendications

1. Centrale électrique intermédiaire (1) comprenant une installation intégrée de gazéification du charbon (2), une partie de centrale à turbine à gaz (5), reliée à l'installation de gazéification du charbon, une partie de centrale à vapeur (6), reliée à l'installation à échangeurs de chaleur pour le gaz brut (3) de l'installation de gazéification du charbon, et une installation de synthèse du méthanol (7), caractérisée en ce que l'installation de synthèse du méthanol (7) est composée de modules (54, 55, 56) montés en parallèle et communique avec la partie de centrale à turbine à gaz par un système central de répartition du gaz purifié (8), qui comprend une installation de stockage intermédiaire (10), dans laquelle passe du gaz purifié et montée en parallèle avec le conduit d'alimentation en gaz purifié (9) et qui est monté en aval, suivant la direction du gaz, de l'installation à échangeurs de chaleur pour le gaz brut (3).

2. Centrale électrique intermédiaire suivant la revendication 1, caractérisée en ce que l'installation de stockage intermédiaire dans laquelle passe du gaz purifié (10) est constituée en installation de régulation et de stockage intermédiaire de façon à maintenir constante la pression dans le conduit d'alimentation en gaz purifié (9), et comporte un réservoir basse pression (47) et un réservoir haute pression (48) qui communiquent entre eux par un compresseur (49) augmentant la pression.

3. Centrale électrique intermédiaire suivant la revendication 1, caractérisée en ce que l'installattion d'échangeurs de chaleur pour le gaz brut (3) comprend trois échangeurs de chaleur (20, 21, 22) dont le premier (20) et le troisième (22) servent à produire de la vapeur et dont le second sert à préchauffer le gaz purifié entrant dans la chambre de combustion (27) de la turbine à gaz (28) de la partie de centrale à turbine à gaz (5).

4. Centrale électrique intermédiaire suivant la revendication 3, caractérisée en ce que les capacités des premier et troisième échangeurs de chaleur (20 et 22) sont telles qu'ils suffisent, lorsque la turbine à gaz (28) est arrêtée et lorsque l'installation de gazéification du charbon (2) fonctionne, à entraîner la turbine à vapeur (36) pour le maintien de l'auto-alimentation en électricité de l'installation de gazéification du charbon (2) et de l'installation de synthèse du méthanol (7).

5. Centrale électrique intermédiaire suivant la revendication 3 ou 4, caractérisée en ce que le troisième échangeur de chaleur (22) est conçu avec une conformation adéquate des surfaces de chauffe de manière à pouvoir absorber la chaleur du gaz brut se produisant en plus lors d'une charge partielle ou d'un arrêt de la turbine à gaz (28).

6. Centrale électrique intermédiaire suivant la revendication 1, caractérisée en ce que le gaz résiduaire de synthèse de l'un au moins des modules (55, 56) de l'installation de synthèse du méthanol (7) peut être retourné au réacteur de synthèse (57, 58) à l'aide d un compresseur de recyclage (63, 64), en passant par un étage d'enrichissement en hydrogène (65, 66).

7. Centrale électrique intermédiaire suivant la revendication 1, caractérisée en ce que le gaz résiduaire de synthèse d'au moins un module (57) de l'installation de synthèse du méthanol (7), qui n'a pas entièrement réagi dans le réacteur de synthèse (67), peut être envoyé en passant par une zone de mélange (52), dans le conduit d'alimentation en gaz purifié (9) menant à la partie de centrale à turbine à gaz (5).

8. Centrale électrique intermédiaire suivant la revendication 6 ou 7, caractérisée en ce que le gaz résiduaire de synthèse d'au moins un module (54) de l'installation de synthèse du méthanol (7), qui n'a pas entièrement réagi, peut être envoyé, pour accélérer la mise en fonctionnement du réacteur de synthèse d'un autre module (55, 56) dans le conduit (61, 62) de recyclage retournant au réacteur de synthèse (57, 58) de cet autre module.

9. Centrale électrique intermédiaire suivant la revendication 1 ou 6, caractérisée en ce que le gaz restant des modules individuels (55, 56) de l'installation de synthèse du méthanol (7), qui a été soutiré dans les conduits de recyclage (61, 62) peut être brûlé dans un générateur de vapeur distinct et la vapeur obtenue peut être envoyée à la partie de centrale à vapeur (6).

10. Centrale électrique intermédiaire suivant la revendication 1 ou 6, caractérisée en ce que le gaz restant des modules individuels (55, 56) de l'installation de synthèse du méthanol (7), qui a réagi dans une grande mesure sous l'effet au recyclage, est brûlé dans une turbine à gaz distincte pour couvrir les besoins propres en énergie électrique de celle-ci.

11. Centrale électrique intermédiaire suivant la revendication 3, caractérisée en ce qu'un dispositif de refroidissement et de réglage (23) refroidi à l'eau, est utilisé pour maintenir constante la température de sortie du gaz brut de l'installation à échangeurs de chaleur pour le gaz brut (3).

12. Centrale électrique intermédiaire suivant la revendication 1, caractérisée en ce qu'au gazéificateur de charbon (11) est associé au moins un compresseur d'air (17, 18) supplémentaire qui est monté en parallèle au compresseur d'air (30) de la partie de centrale à turbine à gaz (5) et par lequel l'alimentation en air de l'installation de fractionnement de l'air (12) en amont du gazéificateur de charbon peut être complétée.

13. Centrale électrique intermédiaire suivant la revendication 12, caractérisée en ce que le compresseur d'air (17, 18) supplémentaire prend en charge, lorsque la partie de centrale à turbine à gaz (5) est arrêtée, l'alimentation de l'installation de gazéification du charbon (2) pour le fonctionnement de l'installation de synthèse du méthanol (7).

14. Centrale électrique intermédiaire suivant la revendication 1, caractérisée en ce qu'un réservoir tampon d'oxygène (13) est prévu entre le gazéificateur de charbon (11) et l'installation de fractionnement d'air (12) qui est en amont du gazéificateur de charbon.

15. Centrale électrique intermédiaire suivant la revendication 1, caractérisée en ce que le conduit pour l'azote de l'installation de fractionnement d'air (12), en amont du gazéificateur de charbon (11), communinue par un réservoir tampon (14) avec le conduit pour le gaz purifié (9) menant au brûleur (27) de la partie de centrale à turbine à gaz (5).